Europäisches Patentamt

European Patent Office

Office européen des brevets

(19)

(11) Numéro de publication : **0 477 114 A1**

# (12) DEMANDE DE BREVET EUROPEEN

(21) Numéro de dépôt : **91420331.0**

(22) Date de dépôt : **17.09.91**

(51) Int. Cl.$^5$ : **A61F 2/28**

(30) Priorité : **21.09.90 FR 9011899**

(43) Date de publication de la demande :
**25.03.92 Bulletin 92/13**

(84) Etats contractants désignés :
**BE CH DE ES FR GB IT LI**

(71) Demandeur : **IMPACT**
**Les Hortensias**
**F-01800 Charnoz (FR)**
(71) Demandeur : **Rigal, Francois**
**4 Route de Lyon**
**F-69450 Saint Cyr au Mont D'or (FR)**
(71) Demandeur : **Roussouly, Pierre**
**41 Rue Boileau**
**F-69006 Lyon (FR)**

(72) Inventeur : **Rigal, François**
**4 Route de Lyon**
**F-69450 Saint Cyr au Mont d'Or (FR)**
Inventeur : **Roussouly, Pierre**
**41 rue Boileau**
**F-69006 Lyon (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

(54) Endoprothèse orthopédique de moignon d'amputation de membre.

(57)   Endoprothèse orthopédique de moignon d'amputation de membre, comprenant une tige (1) destinée à être insérée dans la cavité médulaire de l'os du membre amputé, caractérisée en ce qu'elle comprend en outre :
— une embase (10), destinée à recevoir l'extrémité de la tige (1) et à venir en appui sur la tranche de section corticale du segment osseux sectionné ;
— un corps (20) fixé à l'extrémité de la tige (1), comportant une pluralité d'orifices traversants (21,22) destinés à recevoir les moyens de fixation des tendons et des ligaments du membre amputé.

FIG.2

EP 0 477 114 A1

L'invention concerne une endoprothèse orthopédique de moignon d'amputation de membre.

Si dans la suite de la description, l'invention est plus particulièrement décrite dans son application à l'amputation du fémur, il va de soi qu'elle peut néanmoins être utilisée pour les autres membres ou sections de membre.

Actuellement, le plus généralement, les handicapés d'un membre avec section et perte complète de l'extrémité distale du segment de membre, par exemple du fémur, marchent avec une exoprothèse avec appui au niveau de l'ischion ou avec un appui proximal au niveau du moignon entrainant des troubles de la marche qui sont toujours cause de boiterie. L'appui distal au niveau de l'extrémité inférieure du fut fémoral sectionné, est le plus souvent impossible en raison des douleurs d'appui provoquées par la sensibilité du perioste, et par l'impaction du fémur dans les parties molles du moignon. Cela empêche toute mise en charge satisfaisante qui empêche le moignon de garder une trophicité normale.

On a également suggéré d'allonger le moignon d'amputation par un fixateur externe positionné dans l'os par des broches transcutanées. Cette technique fait appel à un traitement chirurgical long et coûteux, entraînant des risques d'infection, notamment du fait des broches, et dont les résultats sont aléatoires (échecs de l'ordre de la moitié des interventions).

L'invention pallie ces inconvénients. Elle vise une endoprothèse du type en question qui soit facile à mettre en place, moins coûteuse car ne nécessitant pas un traitement chirurgical long, atténuant les risques d'infection et améliorant les bons résultats.

Cette endoprothèse orthopédique de moignon d'amputation de membre, qui comprend une tige destinée à être inserrée dans la cavité médulaire de l'os du membre amputé, se <u>caractérise</u> en ce qu'elle comprend en outre :

– une embase, destinée à recevoir l'extrémité de la tige et à venir en appui sur la tranche de section corticale du segment osseux sectionné ;

– un corps fixé à l'extrémité de la tige, comportant une pluralité d'orifices traversants, destinés à recevoir les moyens de fixation des tendons et des ligaments du membre amputé.

Avantageusement, en pratique :

– la tige présente une section cylindrique et une extrémité arrondie, et comporte soit un filetage à pans coupés arrondis, soit une forme correspondant à la morphologie de la cavité médullaire du segment osseux dans lequel est implantée la tige ;

– l'autre l'extrémité de la tige comporte un emmanchement cylindrique associé à un épaulement également cylindrique, lequel est associé à son tour à une tête légèrement conique ;

– l'embase a la forme d'un chanfrein et présente un logement, complémentaire de l'emmanchement et de l'épaulement de la tige, de manière à coopérer avec celle-ci ;

– le corps a une forme proche de la morphologie anatomique du segment osseux distal manquant, et est recouvert d'un élément souple destiné à faciliter l'adhérence aux tissus périphériques, tels que par exemple un tricot tubulaire en fil polyester ou tout système poreux ou adhésif permettant l'ancrage par fibrose des tissus environnants ;

– le corps présente :

. sur le dessus, des entailles destinées à recevoir une extrémité du tricot tubulaire, bloqué contre l'embase par une rondelle ;

. au centre, un alésage conique ouvert pour coopérer avec la tête conique de la tige, puis un alésage taraudé pour recevoir ultérieurement un extracteur d'embase,

. enfin un trou de lamage, destiné à recevoir l'autre extrémité du tricot tubulaire pincée contre les bords de ce lamage par un organe de maintien ;

– le corps présente au moins deux jeux de deux trous traversants disposés dans deux plans distincts parallèles ;

– les trous traversants sont inclinés ;

– la tige, l'embase et le corps sont en matériau biocompatible (acier, titane, céramique, matière plastique).

La manière dont l'invention peut être réalisée et les avantages qui en décollent ressortiront mieux de l'exemple de réalisation qui suit.

La figure 1 est une représentation vue de côté de la tige caractéristique de l'invention.

La figure 2 représente en coupe l'embase caractéristique de l'invention.

Les figures 3 et 4 montrent respectivement en coupe vu de dessus le corps caractéristique de l'invention.

La figure 5 montre une prothèse complète conforme à l'invention.

La figure 6 montre une autre forme d'exécution de réalisation de la prothèse conforme à l'invention.

Les figures 7 et 8 illustrent une autre forme d'exécution de l'invention.

L'endoprothèse orthopédique de moignon d'amputation de fémur conforme à l'invention détaillée aux figures, comprend tout d'abord une tige désignée par la référence générale (1) formée d'un fût cylindrique (2), dont l'extrémité (3) destinée à être inserrée dans le canal médulaire est arrondie. La base de la tige comporte un emmanchement cylindrique (4) associé à son tour à un épaulement (5) également cylindrique, solidaire d'une tête conique (6) percée en son centre d'un trou taraudé (7). Une entaille (8) permet le vissage de l'ensemble. La tête conique (6) présente un angle au sommet type mors de 5 à 6°.

La longueur du fût (2) varie en fonction de la longueur nécessaire, par exemple de cinquante à deux cent millimètres. De même, le diamètre du fût (2) peut varier en fonction des applications envisagées. En revanche, quelle que soit la longueur ou le diamètre du fût (2), l'emmanchement (4) et l'épaulement (5) ont des dimensions constantes.

Le fût (2) présente un filetage (9) dont le pas de vis présente une section trapézoïdale à pans coupés, dans lequel les raccords sont avantageusement arrondis. Pour obtenir dans le canal médullaire la poussée axiale maximum avec toutefois le minimum d'effet radial, l'angle d'inclinaison de la face supérieure par rapport à la direction du pas de vis est moins incliné que la face inférieure par rapport au même plan de la section transversale du fût (2).

Il va de soi que si dans l'exemple décrit, la tige (1) est filetée (9), elle peut également être ajustée par tout autre moyen connu, tel que collage, ciment ou analogue.

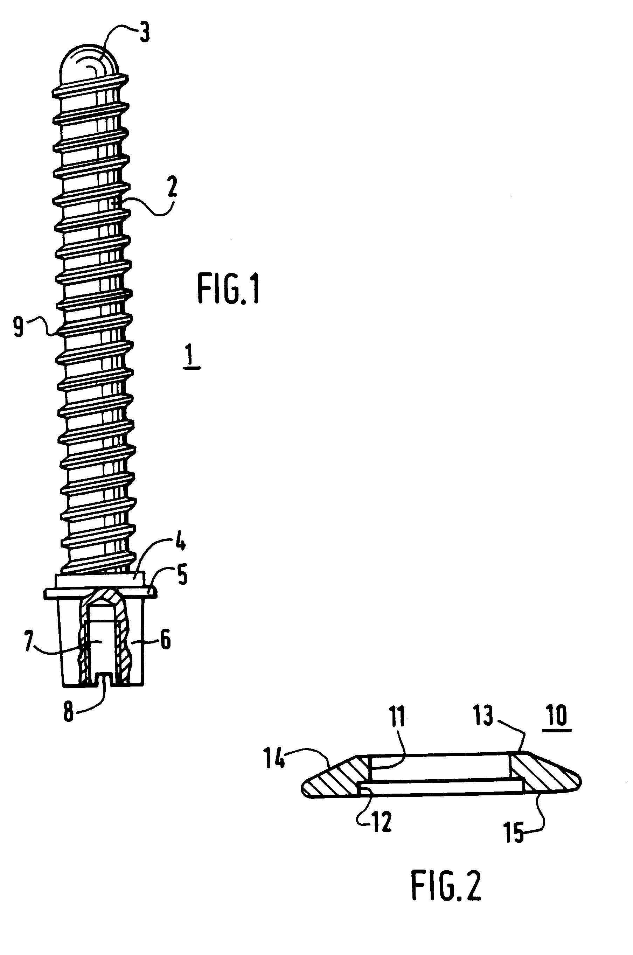

La prothèse comporte ensuite une embase désignée par la référence générale (10) montrée en coupe à la figure 2, qui présente en son centre un premier logement (11) complémentaire de l'emmanchement (4), et un second logement (12) complémentaire de l'épaulement (5). Ces deux logements (11) et (12) sont destinés à coopérer avec (4) et (5). La face supérieure (13) de l'embase vient prendre appui sur la tranche de section corticale du segment osseux sectionné. Cette face d'appui (13) est reliée par un chanfrein (14) à la base (15) de l'embase. Si le plus généralement les logements (11) et (12) ont un diamètre constant, en revanche, le diamètre externe le plus extrême du chanfrein (14) peut varier selon l'os à prolonger.

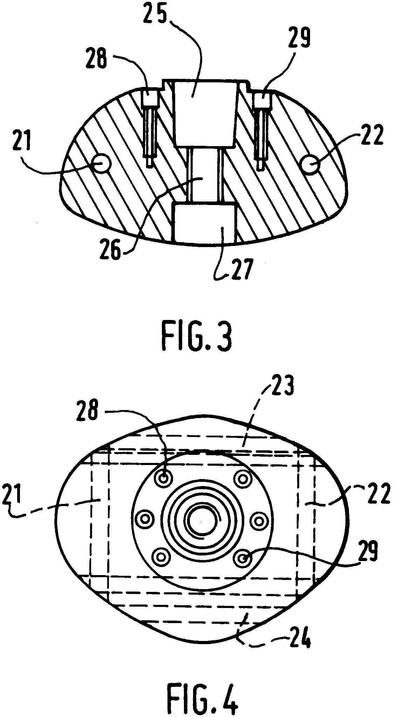

La prothèse selon l'invention comprend ensuite un corps désigné par la référence générale (20) montré en détail aux figures 3 et 4. Ce corps présente tout d'abord une forme proche de la morphologie anatomique du segment osseux distal manquant. Par exemple, dans le cas du fémur, cette forme rappelle celle du condyle fémoral pour l'extrémité distale du fémur. Selon une caractéristique essentielle de l'invention, le corps (20) présente une pluralité de paires d'orifices traversants, respectivement (21,22), destinés à recevoir les moyens de fixation des tendons et des ligaments du membre amputé et plus précisément, à permettre le passage des faisceaux de fils ou de néotendons artificiels. Dans une forme d'exécution montrée aux figures 3 et 4, cette paire d'orifices traversants (21,22) est disposée dans le même plan horizontal. Une seconde paire d'orifices traversants orthogonaux (23,24) peut être disposée dans un plan différent.

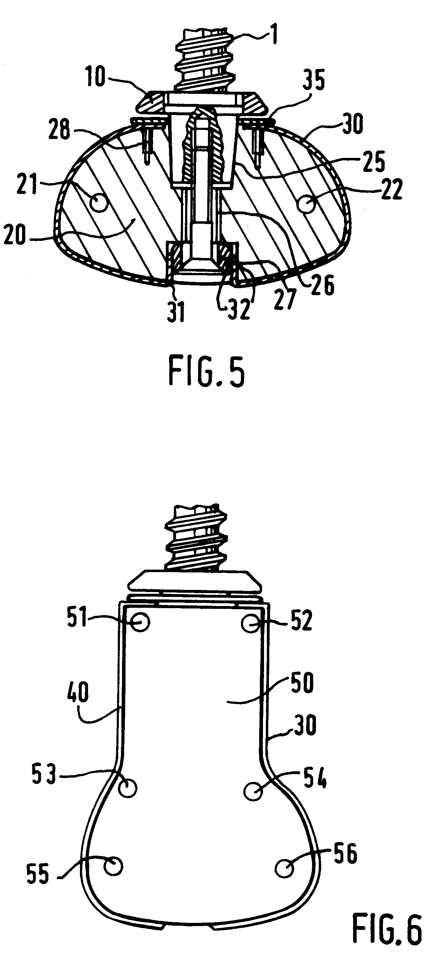

Dans la forme d'exécution montrée à la figure 6, le corps désigné ici par la référence générale (50), présente dans chaque section trois paires d'orifices traversants, respectivement (51 à 56). Ici (figure 6), le corps (50) a une forme générale de poire et est particulièrement destiné à répartir les efforts d'appui du membre en rappelant l'anatomie des surfaces articulaires manquantes (condyles).

Ce corps (20) présente en son centre de haut en bas, respectivement un alésage conique ouvert (25) destiné à coopérer avec la tête conique (6) de la tige et dont l'emboitement est assuré par un cone morse, puis un alésage taraudé (26) destiné à recevoir ultérieurement un extracteur d'embase, et enfin un trou de lamage (27) débouchant sur la face inverse. La face supérieure du corps (20) comporte également des trous taraudés, respectivement (28,29), par exemple au nombre de six régulièrement décalées angulairement.

Selon une autre caractéristique essentielle de l'invention (voir figure 4), le corps (20,50) est recouvert d'un tricot tubulaire en fil de polytéréphtalate d'éthylène glycol (polyester) (30) ou (40), dont une extrémité est pincée dans les trous taraudés (28,29) et dont l'autre extrémité (31) est inserrée dans le trou de lamage (27) pour être pincée avec un clips (32), une bague de maintien ou analogue. Une rondelle de serrage (35) (voir figure 5), assure un meilleur maintien et un meilleur blocage de l'extrémité supérieure du tricot tubulaire caractéristique (30).

Les tricots tubulaires d'adhérence (30,50) favorisent l'adhérence du corps (20) aux tissus périphériques (muscles, aponevroses, tissus adipeux, etc. ), ce qui évite l'instabilité de ces tissus par rapport à l'endoprothèse. En outre, le tricot (30) permet la fixation de points de suture et comme déjà dit, également une sclérose des tissus.

En pratique, tous les éléments de la prothèse à savoir : tige (1), embase (10), corps (20), rondelle (35), sont en un alliage métallique, notamment à base de titane, en acier chrome/cobalt ou équivalent, sous réserve d'être bio-compatible.

Comme déjà dit, si l'invention est décrite dans son application au moignon de fémur, ce type d'endoprothèse peut également trouver d'autres applications, par exemple pour les moignons d'amputation du tibia, du pied, de l'humérus, de l'avant-bras ou même de la main, sous réserve pour le corps (20,50) de reproduire la forme anatomique de l'extrémité du segment du membre amputé et du moignon, pour faciliter comme déjà dit l'appui et l'accrochage de la prothèse.

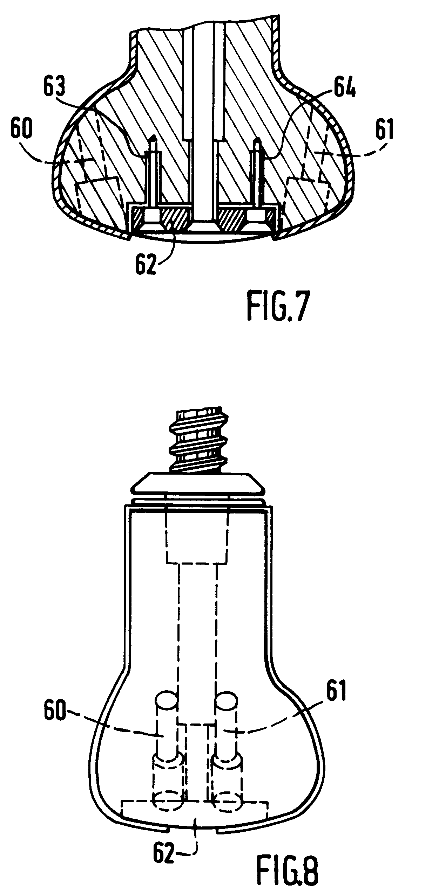

Les figures 7 et 8 illustrent de manière simplifiée une forme d'exécution préférée de l'invention dans laquelle les trous taraudés caractéristiques destinés à recevoir les moyens de fixation, désignés par les références (60) et (61), sont légèrement inclinés par rapport à la verticale. Dans cette forme d'exécution, le clips de maintien (32) est remplacé par une bague de blocage (62) bloquant l'extrémité de la tresse, maintenue en place dans le trou de lamage (27) par des vis (63) et (64).

Les endoprothèses de moignon d'amputation de membre conforme à l'invention, présentent de nombreux avantages par rapport à ce qui était commercialisé à ce jour. On peut citer :

– tout d'abord, le fait que grâce à l'embase, on obtient un appui terminal du moignon dans une exhoprothèse conventionnelle ;

– la morphologie du corps (20,50) qui facilite l'ancrage de l'exhoprothèse en phase de décharge, lors de la marche ;

– la facilité et la rapidité de mise en place, qui ne nécessite pas des traitement chirurgicaux longs, affaiblissants et coûteux, et ce qui avantageusement diminue la durée d'hospitalisation ;

– la possibilité de régler l'embase en rotation par rapport au membre, lors de la pose et avant le blocage de l'emmanchement conique ;

– le fait que l'on évite ainsi les excroissances osseuses au niveau de la tranche de section type osteophyte ;

– la possibilité de rallonger les moignons, ce qui entraîne ainsi la formation d'un bras de levier favorable pour l'utilisateur.

On améliore donc ainsi les performances fonctionnelles des moignons d'amputation dans un but de prothétisation externe par tout moyen conventionnel. En outre, ces endoprothèses permettent de fixer les éléments musculaires et ligamentaires qui, le plus généralement, à ce jour, sont laissés libres par l'amputation ou fixés entre eux.

**Revendications**

**1/** Endoprothèse orthopédique de moignon d'amputation de membre, comprenant une tige (1) destinée à être inserrée dans la cavité médulaire de l'os du membre amputé, _caractérisée_ en ce qu'elle comprend en outre :

– une embase (10), destinée à recevoir l'extrémité de la tige (1) et à venir en appui sur la tranche de section corticale du segment osseux sectionné ;

– un corps (20, 50) fixé à l'extrémité de la tige (1), comportant une pluralité d'orifices traversants (21,22, 23,24 ; 51-56 ; 60,61) destinés à recevoir les moyens de fixation des tendons et des ligaments du membre amputé.

**2/** Endoprothèse selon la revendication 1, caractérisée en ce que la tige (1) présente un fût (2) cylindrique et une extrémité (3) arrondie, et comporte un filetage (9) à pans coupés arrondis.

**3/** Endoprothèse selon la revendication 1, caractérisée en ce que la tige (1) présente un fût (2) cylindrique et une extrémité (3) arrondie, et est de forme correspondant à la morphologie de la cavité médullaire du segment osseux dans laquelle ladite tige (1) est implantée.

**4/** Endoprothèse selon la revendication 1, caractérisée en ce que l'autre extrémité de la tige (1) comporte un emmanchement (4) cylindrique, associé à un épaulement (5) également cylindrique, lequel est fixé à une tête conique (6).

**5/** Endoprothèse selon la revendication 1, caractérisée en ce que l'embase (10) présente un premier logement (11) complémentaire de l'emmanchement (4), associé à un second logement (12) complémentaire de l'épaulement (5), de manière à coopérer avec eux, et présente sur le dessus (13) une face d'appui raccordée par un chanfrein (14) à la base (15).

**6/** Endoprothèse selon la revendication 1, caractérisé en ce que le corps (20,50) a une forme proche de la morphologie anatomique du segment osseux distal manquant.

**7/** Endoprothèse selon les revendications 1 à 6, caractérisée en ce que le corps (20,50) est recouvert d'un élément souple (30) destiné à faciliter l'adhérence aux tissus périphériques.

**8/** Endoprothèse selon la revendication 7, caractérisée en ce que l'élément souple (30) est un tricot tubulaire en fil polyester.

**9/** Endoprothèse selon la revendication 1, caractérisée en ce que le corps (20,50) présente :

. sur le dessus, des trous taraudés (28,29) destinées à recevoir une extrémité du tricot tubulaire (30), bloquée contre l'embase par une rondelle (35) ;

. en son centre, le coprs (20) est percé dans l'ordre d'un alésage conique ouvert (25) destiné à coopérer avec la tête conique (6) de la tige, continué par un alésage taraudé (26) et terminé par trou de lamage (27), destiné à recevoir l'autre extrémité du tricot tubulaire (30) bloquée dans ce trou (27) par un élément de maintien (32).

**10/** Endoprothèse selon l'une des revendications 1 à 9, caractérisé en ce que le corps (20,50) présente au moins deux jeux (21-24 ; 51-56), de deux trous traversants disposés dans des plans parallèles.

FIG.1

FIG.2

FIG.3

FIG.4

FIG.5

FIG.6

FIG.7

FIG.8

**Office européen des brevets**

## RAPPORT DE RECHERCHE EUROPEENNE

Numero de la demande

EP 91 42 0331

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-3 439 993 (HEIMKE)<br>* page 3, ligne 8 - page 4, ligne 15; figures *<br>--- | 1,2 | A61F2/28 |
| A | GB-A-2 231 271 (KRASNODARSKI)<br>* page 3, ligne 24 - page 6, ligne 9; figures *<br>--- | 1-5 | |
| A | US-A-4 158 895 (FROSCH)<br>--- | - | |
| A | FR-A-2 306 672 (GLASROCK PRODUCTS)<br>--- | - | |
| A | DE-A-3 132 543 (WEBER)<br><br>----- | - | |

**DOMAINES TECHNIQUES RECHERCHES (Int. Cl.5)**

A61F

Le présent rapport a été établi pour toutes les revendications

| Lieu de la recherche | Date d'achèvement de la recherche | Examinateur |
|---|---|---|
| LA HAYE | 02 JANVIER 1992 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)